(19) Europäisches Patentamt
European Patent Office
Office européen des brevets

(11) Numéro de publication: **0 294 258**
**A1**

# (12) DEMANDE DE BREVET EUROPEEN

(21) Numéro de dépôt: **88401149.5**

(22) Date de dépôt: **11.05.88**

(51) Int. Cl.⁴: **C 07 C 109/12**
C 07 C 133/00, A 61 K 31/75,
A 61 K 31/325, A 61 K 31/27

(30) Priorité: **14.05.87 FR 8706801**

(43) Date de publication de la demande:
**07.12.88 Bulletin 88/49**

(84) Etats contractants désignés:
**AT BE CH DE ES FR GB GR IT LI LU NL SE**

(71) Demandeur: **Société anonyme: LES LABORATOIRES MERAM**
**4 Bld Malesherbes**
**F-75008 Paris (FR)**

(72) Inventeur: **Miocque, Marcel**
**Résidence Baudelaire, 9, rue de Malabry**
**F-92350 Le Plessis Robinson (FR)**

**Binet, Pierre**
**Hôpital Ste Anne, 1, rue Cabanis**
**F-75014 Paris (FR)**

**Galons, Hervé**
**7, rue des Wallons**
**F-75013 Paris (FR)**

(74) Mandataire: **Gillard, Marie-Louise et al**
**Cabinet Beau de Loménie 55, Rue d'Amsterdam**
**F-75008 Paris (FR)**

## (54) Dérivés d'hydrazine, procédé d'obtention et compositions pharmaceutiques les contenant.

(57) La présente invention concerne des dérivés d'hydrazine répondant à la formule :

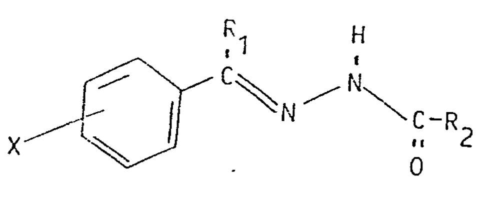

dans laquelle :
X est un alkyle en $C_1$-$C_4$, le groupe trifluorométhyle ou un halogène choisi parmi le chlore (en position ortho ou méta) le fluor ou le brome,
$R_1$ est un groupe alkyle en $C_1$-$C_4$ ;
$R_2$ représente :
- l'hydrogène ;
- un groupe alkyle ramifié en $C_3$-$C_4$ .
- un groupe alcoxy en $C_1$-$C_4$, de préférence éthoxy ;
- un groupe de formule $CR_3R_4R_5$ dans lequel :
  . $R_3$ représente un groupe en $OR_6$, $SR_6$ ou $NR_7R_8$ dans lesquels:
  $R_6$ est l'hydrogène ou un alkyle $C_1$-$C_4$ .
  $R_7$ et $R_8$ représentent l'hydrogène, un groupe alkyle en $C_1$-$C_4$, un groupe acyle en $C_2$-$C_4$, un groupe benzoyle ou bien $R_7$ et $R_8$ ensemble avec l'atome d'azote auquel ils sont liés forment un groupe hétérocyclique à 5 ou 6 chaînons ;
  . $R_4$ et $R_5$ sont identiques et représentent l'hydrogène,ou un groupe alkyle en $C_1$-$C_4$ ou bien $R_4$ est l'hydrogène et $R_5$ représente soit un groupe alkyle en $C_1$ - $C_4$ éventuellement substitué par un groupe hydroxy, soit un groupe phényle ; ou bien
  . $R_3$, $R_4$, et $R_5$, ensemble avec l'atome de carbone auquel ils sont liés, forment un groupe amido ;
- un groupe phényl éventuellement substitué par un groupe hydroxy ;
- un groupe hétérocycle à 5 ou 6 chaînons et les sels pharmaceutiquement acceptables desdits composés.

EP 0 294 258 A1

## Description

**Dérivés d'hydrazine, procédé d'obtention et compositions pharmaceutiques les contenant.**

La présente invention concerne des nouveaux dérivés d'hydrazine qui se sont révélés utiles notamment comme agents antihypertenseurs. Elle a également pour objet un procédé pour leur obtention ainsi que les compositions pharmaceutiques les contenant.

On connaît déjà des dérivés d'hydrazine ayant des propriétés pharmacologiques. Ces dérivés sont par exemple les esters d'acides carbaziques substitués décrits dans le brevet U.S. 3 867 425, qui répondent à la formule :

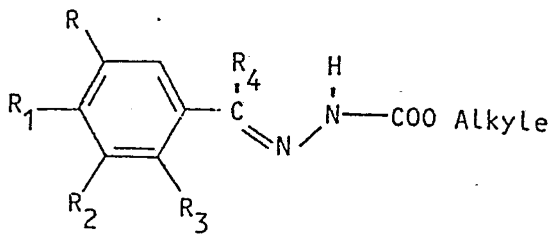

I

dans laquelle R et $R_3$ sont notamment l'hydrogène, $R_1$ et $R_2$ sont par exemple l'hydrogène ou le chlore et $R_4$ est l'hydrogène ou un groupe alkyle. Ces composés sont utiles pour le traitement des affections rhinovirales.

On peut également citer les brevets U.S. 3 746 703, 3 836 580 et 3 859 281 qui décrivent des 2,6-dichlorobenzylidènehydrazides. Ces composés présentent une activité anti-dépressive et le plus souvent également une activité antihypertensive. Ces composés répondent à la formule :

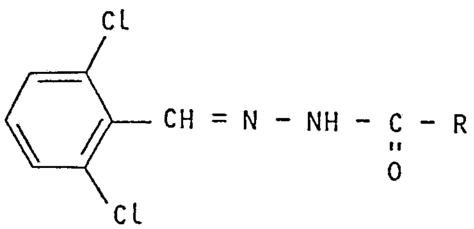

II

dans laquelle R est par exemple un alkyle, un hydroxyalkyle, un alcényle, un alcynyle, ou un groupe hétérocyclique.

Ces composés semblent se situer dans un courant de recherches liées à la "Clonidine", dont les deux atomes de chlore en position 2 et 6 paraissent jouer un rôle déterminant dans l'activité.

D'autres dérivés d'hydrazine présentant des propriétés pharmacologiques ont également été décrits ; ils répondent à la formule :

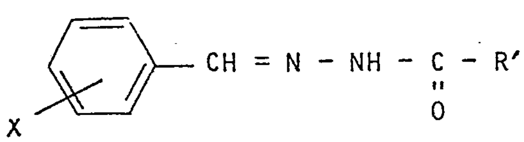

dans laquelle :

- X est un halogène, tel que le chlore ou le brome ;
- R′ est notamment un groupe alkyle substitué par un groupe amino tertiaire, un groupe hétérocyclique, un groupe phényle di- ou trisubstitué ou un groupe amino.

Au sujet de ces composés on peut se référer aux ouvrages ci-après :

- JOURNAL OF MEDICINAL CHEMISTRY, vol. 14, n° 10, 1971 p. 1017-1020
- IL FARMACO ED. SC., vol. 36, fasc. 4, 1981 p. 269-273.
- IL FARMACO ED. SC., vol. 33, fasc. 12, 1978 p. 963-971 ;
- JOURNAL OF MEDICINAL CHEMISTRY, vol. 20, n° 11, 1977, p. 1520-1521.

On a maintenant trouvé une nouvelle famille de dérivés d'hydrazine qui présentent des propriétés d'antihypertenseurs et une toxicité moindre que les composés voisins décrits précédemment.

Les dérivés d'hydrazine selon l'invention répondent à la formule :

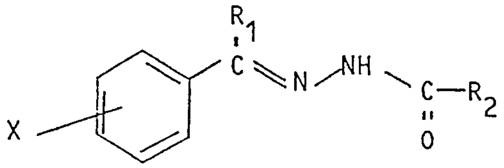 IV

dans laquelle
X est un alkyle en $C_1$-$C_4$, le groupe trifluorométhyle ou un halogène choisi parmi le chlore (en position ortho ou méta) le fluor ou le brome,
$R_1$ est un groupe alkyle en $C_1$-$C_4$ ;
$R_2$ représente :
- l'hydrogène ;
- un groupe alkyle ramifié en $C_3$ - $C_4$ ;
- un groupe alcoxy en $C_1$ - $C_4$, de préférence éthoxy ;
- un groupe de formule $CR_3R_4R_5$ dans lequel :
  . $R_3$ représente un groupe en $OR_6$, $SR_6$ ou $NR_7R_8$ dans lesquels :
  $R_6$ est l'hydrogène ou un alkyle $C_1$-$C_4$ ;
  $R_7$ et $R_8$ représentent l'hydrogène, un groupe alkyle en $C_1$-$C_4$, un groupe acyle en $C_2$ - $C_4$, un groupe benzoyle ou bien $R_7$ et $R_8$ ensemble avec l'atome d'azote auquel ils sont liés forment un groupe hétérocyclique à 5 ou 6 chaînons ;
  . $R_4$ et $R_5$ sont identiques et représentent l'hydrogène, ou un groupe alkyle en $C_1$-$C_4$ ou bien $R_4$ est l'hydrogène et $R_5$ représente soit un groupe alkyle en $C_1$ - $C_4$, éventuellement substitué par un groupe hydroxy, soit un groupe phényle ; ou bien
  . $R_3$, $R_4$ et $R_5$, ensemble avec l'atome de carbone auquel ils sont liés, forment un groupe amido ;
- un groupe phényle éventuellement substitué par un groupe hydroxy
- un groupe hétérocyclique à 5 ou 6 chaînons ;

A titre d'exemples de groupes hétérocycliques, on peut citer les groupes morpholinyle, pipéridyle, pyridyle ou pyrrolidonyle.

La présente invention concerne également les sels pharmaceutiquement acceptables des composés de formule IV lorsqu'ils existent. Ces sels sont obtenus selon des procédés classiques de salification avec des acides pharmaceutiquement acceptables. Le chlorhydrate est le sel particulièrement préféré.

L'invention concerne également les isomères E et Z autour de la liaison C=N.

On notera que les composés de l'invention sont caractérisés par les points suivants :
- la substitution du carbone benzylidènique par un groupe alkyle inférieur ($R_1$) ;
- la mono- substitution du groupe phényle ;
- la position de l'hétéroatome du radical $R_2$, lorsqu'il existe ($R_3$ est $OR_6$, $SR_6$ ou $NR_7R_8$), sur le carbone en du groupe CO.

On notera qu'une classe particulière des composés de l'invention est celle des composés de formule IV dans laquelle $R_2$ est le groupe -$CR_3R_4R_5$ tel que défini ci-dessus et notamment les composés de formule IV dans laquelle $R_2$ est le groupe -$CR_3R_4R_5$, X est un halogène et $R_1$ est le groupe méthyle.

L'invention concerne également un procédé pour préparer les dérivés d'hydrazine de formule IV qui consiste à obtenir :
- au cours d'une première étape, une acylhydrazine de formule (I)

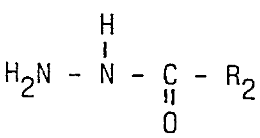 (1)

où $R_2$ est tel que défini ci-dessus à partir de l'hydrate d'hydrazine et d'un ester de formule (2) $R_2$ - COOR dans laquelle $R_2$ est tel que défini précédemment et R représente un groupe alkyle ;
- puis à faire réagir ensuite le produit (1) avec une arylalkylcétone de formule (3) :

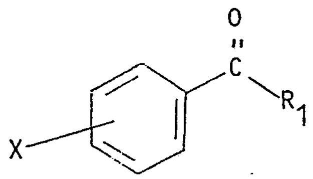

(3)

où $R_1$ et X sont tels que définis précédemment.

Les deux étapes du procédé sont avantageusement réalisées au reflux en milieu alcoolique, par exemple dans l'éthanol ou dans un mélange éthanol-eau. Les produits de l'invention sont ensuite récupérés du milieu réactionnel par des moyens classiques à la portée de l'homme de l'art, et éventuellement à les transformer en leurs sels pharmaceutiquement acceptables, selon des procédés classiques à la portée de l'homme de l'art.

Les produits de l'invention ont des toxicités faibles rapportées aux activités pharmacologiques intéressantes qu'ils manifestent, lesquelles sont principalement des propriétés antihypertensives, combinées avec des propriétés sédatives, analgésiques et anti-inflammatoires.

Les composés selon l'invention peuvent donc être utilisés en thérapeutique, notamment pour le traitement des hypertensions.

L'invention concerne également les compositions pharmaceutiques contenant à titre de principe actif un dérivé d'hydrazine de formule (IV) en combinaison avec un véhicule pharmaceutiquement acceptable.

Les compositions selon l'invention peuvent se présenter sous la forme de compositions orales (comprimés ou gélules par exemple) ou sous la forme de compositions injectables.

Pour le traitement de l'hypertension, on a trouvé généralement que des doses allant de 5 à 25 mg par jour étaient appropriées.

Les exemples suivants illustrent l'invention sans toutefois en limiter la portée.

Exemple 1

Synthèse de l'(hydroxy-2 isobutyroyl)-1 (orthochlorophényl-1 éthylidène-1)-2 hydrazine dénommé ci-après : produit MP 440

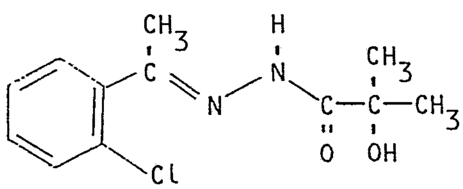

a) Dans un erlenmeyer muni d'un système d'agitation magnétique et surmonté d'un réfrigérant sont introduits successivement 100 ml d'éthanol, 2 g d'hydrate d'hydrazine (0,04 mole) et 5 g d'hydroxy-2 isobutyrate d'éthyle (0,038 mole). Le mélange réactionnel est porté au reflux pendant 3 h. Après refroidissement, la solution est traitée à l'évaporateur rotatif jusqu'à obtention d'un solide qui est filtré et lavé à l'éthanol froid puis à l'éther. Rdt % = 90 PF = 98°C

b) Dans un erlenmeyer muni d'un système d'agitation magnétique et surmonté d'un réfrigérant sont introduits successivement 100 ml d'éthanol, 3,8 g (0,032 mole) d'(hydroxy-2 isobutyroyl)-hydrazine et 5 g d'ortho-chloroacétophénone (0,032 mole). Le mélange est porté au reflux durant 3 h. Après refroidissement la solution est concentrée à l'évaporateur rotatif puis filtrée. Le précipité est recristallisé dans l'éthanol.

Rdt % = 70 PF = 128°C

$^1$H RMN $DMSOd_6$ : ppm : 1,4 (s, 6H, $2CH_3$) ; 2,25 (s, 3H, $CH_3$-C=N) ; 5,8 (éch. $D_2O$, 1H, OH) ; 7,5 (m, 4H, aro.) ; 10,1 (éch. $D_2O$, 1H, NH).

Isomères E et Z du produit MP 440

Le produit brut MP 440 a été analysé par chromatographie sur plaque de silice MERCK 60 F 254 avec la butanone comme solvant de migration. L'observation en UV (254 nm) a révélé l'existence de deux isomères dénommés ci-après : MP 440E et MP 440Z.

MP 440E de Rf : 0,8 et MP 440Z de Rf : 0,7.

La séparation des deux isomères géométriques a été effectuée en deux étapes. Dans une première étape, la recristallisation dans l'éthanol ou la butanone a donné des cristaux de MP 440E. La concentration des eaux mères a permis de recueillir un nouveau précipité d'isomères Z + E.

Les eaux mères qui contenaient alors un mélange des isomères E/Z (environ 40/60) ont été traitées par

chromatographie sur colonne de silice MERCK SILICA GEL 60 avec un mélange éluant toluène-butanone 50/50. Le MP 440E a été récupéré avant l'isomère Z.

Les isomères diffèrent :

1) par leur point de fusion : F = 146° C pour l'isomère Z et 160° C pour l'isomère E.

2) par leur spectre infrarouge (KBr)

MP 440E : 1685 $cm^{-1}$ ; 3100 $cm^{-1}$ (large) ; 3365 $cm^{-1}$ (fine) ; 3440 $cm^{-1}$ (large).

MP 440Z : 1680 $cm^{-1}$ ; 3260 $cm^{-1}$ (large) ; 3340 $cm^{-1}$ (fine) ; 3430 $cm^{-1}$ (large).

3) par leur spectre de RMN$^1$H ($CDCl_3$, TMS)

| | 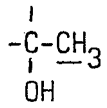 | 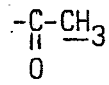 | Aromatiques |
|---|---|---|---|
| MP 440Z | 1,40 | 2,40 | 7,20-7,61 |
| MP 440E | 1,52 | 2,35 | 7,30-7,55 |

4) par leur spectre de RMN$^{13}$C ($CDCl_3$, TMS)

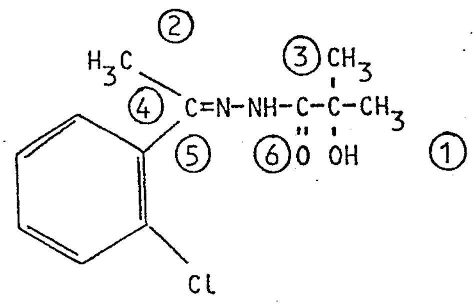

| | 1 | 2 | 3 | 4 | 5 | 6 |
|---|---|---|---|---|---|---|
| MP 440Z | 29,2 | 25,6 | 73,1 | 133,2 | 152,9 | 172,1 |
| MP 440E | 29,2 | 25,6 | 73,6 | 131,5 | 153,96 | 173,0 |

En opérant selon le mode opératoire décrit à l'exemple 1 ou selon des variantes de mise en oeuvre indiquées ci-après, on a préparé les composés 2 à 36 du tableau I ci-après ; les spectres RMN de ces composés sont indiqués ci-après ainsi que les composés de départ mis en oeuvre pour leur obtention

TABLEAU I

| Ex. | X | $R_1$ | $R_2$ | Rende-ment | Point de fusion (solvant de recristallisation) |
|---|---|---|---|---|---|
| 2 | o-Cl | $-CH_3$ | 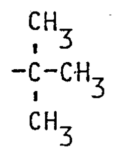 | 73 % | 121°C (éthanol) |
| 3 | o-Cl | $-CH_3$ | 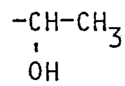 | 62 % | 101°C (éthanol) |
| 4 | o-Cl | $-CH_3$ | $-CH_2OH$ | 80 % | 148°C (éthanol) |
| 5 | o-Cl | $-CH_3$ | 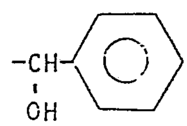 | 65 % | 156°C (éthanol) |
| 6 | o-Cl | $-CH_3$ | $-CH_2-O-CH_3$ | 66 % | 89°C (éthanol) |
| 7 | o-Cl | $-CH_3$ | $-CH_2-NH_2$ | 95 % | 110°C (éthanol) |
| 8 | o-Cl | $-CH_3$ | $-CH_2-S-CH_3$ | 72 % | 150°C (éthanol) |
| 9 | o-Cl | $-CH_3$ | $-CO-NH_2$ | 82 % | 175°C (éthanol) |
| 10 | o-Cl | $-CH_3$ | 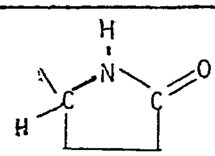 | 65 % | 119°C (éthanol-eau) |
| 11 | o-Cl | $-CH_3$ | 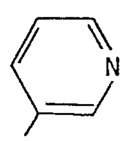 | 62 % | 154°C (éthanol) |
| 12 | o-Cl | $-CH_3$ | $-H$ | 79 % | 138°C (éthanol) |
| 13 | p-F | $-CH_3$ | $-CH_2-NH_2$ | 72 % | chlorhydrate (méthanol) > 260°C |

TABLEAU I (suite)

| Ex. | X | $R_1$ | $R_2$ | Rende-ment | Point de fusion (solvant de recristallisation) |
|---|---|---|---|---|---|
| 14 | m-$CF_3$ | $-CH_3$ | $CH_3$<br>$-C-CH_3$<br>OH | 68 % | 128°C (acétate d'éthyle) |
| 15 | o-Cl | $-CH_3$ | 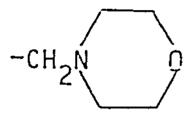 | 79 % | 153 (acétate d'éthyle) |
| 16 | o-Cl | $-C_2H_5$ | $CH_3$<br>$-C-CH_3$<br>OH | 58 % | 141°C (éthanol) |
| 17 | o-F | $-CH_3$ | $-CH_2OH$ | 82 % | 131°C (éthanol) |
| 18 | m-Cl | $-CH_2-CH_3$ | $CH_3$<br>$-C-CH_3$<br>OH | 68 % | 112°C (éthanol) |
| 19 | o-Cl | $-CH_3$ | $-CH_2-NHCOCH_3$ | 83 % | 188-190°C (éthanol) |
| 20 | o-F | $-CH_3$ | $CH_3$<br>$-C-CH_3$<br>OH | 71 % | 142°C (éthanol) |
| 21 | p-F | $-CH_3$ | $CH_3$<br>$-C-CH_3$<br>OH | 58 % | 134°C (éthanol) |
| 22 | o-Br | $-CH_3$ | $CH_3$<br>$-C-CH_3$<br>OH | 69 % | 139°C (éthanol) |

TABLEAU I (suite)

| Ex. | X | $R_1$ | $R_2$ | Rendement | Point de fusion (solvant de recristallisation) |
|---|---|---|---|---|---|
| 23 | o-Br | $-CH_3$ | 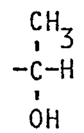 | 62 % | 115°C (éthanol) |
| 24 | o-Br | $-CH_3$ | $-CH_2OH$ | 70 % | 160°C (éthanol) |
| 25 | p-$CF_3$ | $-CH_3$ | 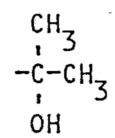 | 69 % | 160°C acétate d'éthyle) |
| 26 | o-Cl | $-C_4H_9$ | 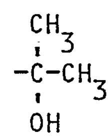 | 78 % | 199°C (acétate d'éthyle) |
| 27 | o-F | $-CH_3$ | 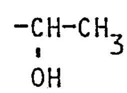 | 52 % | 109°C (éthanol) |
| 28 | m-Cl | $-CH_3$ | 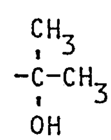 | 68 % | 136°C (éthanol) |
| 29 | m-Cl | $-CH_3$ | 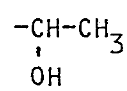 | 61 % | 114°C (éthanol) |
| 30 | o-$CH_3$ | $-CH_3$ | 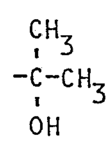 | 65 % | 103°C (éthanol) |

TABLEAU I (suite)

| Ex. | X | $R_1$ | $R_2$ | Rendement | Point de fusion (solvant de recristallisation) |
|---|---|---|---|---|---|
| 31 | o-Cl | $-CH_3$ | $-CH-CH_2OH$<br>$NH_2$ | 71 % | 120-123°C (acétate d'éthyle) |
| 32 | p-$CH_3$ | $-CH_3$ | $-CH_2-NH_2,HCl$ | 58 % | > 260°C (éthanol) |
| 33 | o-Cl | $-CH_3$ | 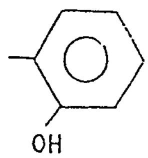 | 71 % | 216°C (éthanol) |
| 34 | m-$CH_3$ | $-CH_3$ | $CH_3$<br>$-C-CH_3$<br>OH | 59 % | 126°C (éthanol) |
| 35 | o-Cl | $-CH_3$ | 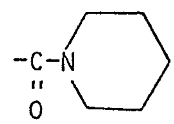 | 73 % | 141°C (éthanol) |
| 36 | o-Cl | $-CH_3$ | $CH_2OH$<br>$-CH-NH-C-C_6H_5$<br>O | 88 % | 183°C-187°C (éthanol) |

Exemple 2

La (triméthyl-2,2,2 acétyl)-1 (orthochlorophényl-1 éthylidène-1)-2 hydrazine a été préparée à partir de triméthyl-2,2,2 acétylhydrazine et de chloro-2′ acétophénone.
RMN$^1$H DMSO$d_6$ : ppm : 1,2 (s, 9H, $3CH_3$) ; 2,25 (s, 3H, $CH_3$-C=H) ; 7,5 (m, 4H aro.) ; 10,2 (éch. $D_2O$, 1H, NH).

Exemple 3

L'(hydroxy-2 propanoyl)-1 (orthochlorophényl-1 éthylidène-1)-2 hydrazine a été préparée selon l'exemple 1 avec une variante consistant à dissoudre, en vue du deuxième stade de la synthèse, l'hydrazide dans 50 ml d'éthanol-eau (50/50) avant mélange avec la chloro-2′ acétophénone.
RMN$^1$H DMSO$d_6$ : ppm : 1,3 (d, 3H, $CH_3$) ; 2,25 (s, 3H, $CH_3$-C=N) ; 4,2 (m, 1H, CH-OH) ; 5,6 (éch. $D_2O$, 1H, OH) ; 7,5 (m, 4H, aro.) ; 10,0 (éch. $D_2O$, 1H, NH).

Exemple 4

L'(hydroxy-2 acétyl)-1 (orthochlorophényl-1 éthylidène-1)-2 hydrazine a été préparée selon l'exemple 1 à partir de l'(hydroxy-2 acétyl) hydrazine et de la chloro-2′ acétophénone.
RMN$^1$H DMSO$d_6$ : ppm : 2,25 (s, 3H, $CH_3$-C=N) ; 4,2 (s, 2H, $CH_2$) ; 5,4 (éch. $D_2O$, 1H, OH) ; 7,5 (m, 4H, aro.) ; 10,4 (éch. $D_2O$, 1H, NH).

Exemple 5

Synthèse de l'(hydroxy-2 phényl-2 acétyl)-1 (orthochlorophényl-1 éthylidène-1)-2 hydrazine.

5.1 Dans un erlenmeyer muni d'un système d'agitation magnétique et d'un réfrigérant on a introduit 30 ml d'éthanol absolu, 0,1 mole d'hydroxy-2 phényl-2 acétate d'éthyle puis 0,105 mole d'hydrazine. Le mélange a été porté au reflux de l'éthanol durant 3 h, puis refroidi à 0°C. L'hydrazide qui a précipité a été séparé par filtration, puis lavé à l'éthanol froid et à l'éther.

5.2 L'(hydroxy-2 phényl-2) acétylhydrazide a été ensuite condensé avec la chloro-2′ acétophénone comme il est indiqué à l'exemple 1.

RMN$^1$H DMSO$d_6$ : ppm : 2,25 (s, 3H, $CH_3$-C=N) ; 5,05 (s, 1H, $CH-\underset{\underset{O}{\|}}{C}$) ; 7,40 (m, 9H, aro.) ; 7,8 (éch. $D_2O$, 1H, OH) ; 10,8 (éch. $D_2O$, 1H, NH).

Exemple 6

Synthèse de la (méthoxy-2 acétyl)-1 (orthochlorophényl-1 éthylidène-1)-2 hydrazine.

Elle a été réalisée à partir du (méthoxy-2 acétyl) hydrazide et de la chloro-2′ acétophénone, selon les indications de l'exemple 5.

RMN$^1$H DMSO$d_6$ : ppm : 2,25 (s, 3H, $CH_3$-C=N) ; 3,5 (s, 3H, $CH_3$-O-) ; 4,4 (s, 2H, $CH_2$) ; 7,4 (m, 4H, aro.) ; 9,8 (éch. $D_2O$, 1H, NH).

Exemple 7

Synthèse de l'(amino-2 acétyl)-1 (orthochlorophényl-1 éthylidène-1)-2 hydrazine.

7.1. On a chauffé durant une heure è 90°C le mélange de 0,10 mole de glycinate d'éthyle (chlorhydrate) et de 0,10 mole d'hydrate d'hydrazine. Après refroidissement, on a ajouté 100 ml d'éther puis on a filtré après trituration.

7.2. L'hydrazide précédent (0,05 mole), solubilisé dans l'éthanol-eau (50/50) a été mis à réagir avec la chloro-2′ acétophénone selon l'exemple 1.

RMN$^1$H DMSO$d_6$ : ppm : 2,25 (s, 3H, $CH_3$-C=N) ; 3,8 (s, 2H, $CH_2$) ; 7,5 (m, 4H, aro.) ; 8,8 (éch. $D_2O$, 3H, $NH_3^+$), 10,8 (éch. $D_2O$, 1H, NH).

Le composé obtenu à l'état de chlorhydrate a été transformé en base selon le protocole suivant : 0,02 mole du chlorhydrate d'aminoacétylhydrazidone ont été mis en suspension dans 200 ml de dichlorométhane. On a fait passer bulle à bulle un courant d'ammoniac dans la suspension durant 5 h, à température ambiante, sous agitation. On a filtré et, par évaporation du filtrat, on a recueilli l'aminoacétylhydrazidone.

RMN$^1$H DMSO$d_6$ : ppm : 2,25 (s, 3H, $CH_3$-C=N) ; 3,6 (s, 2H, $CH_2$) ; 4,5 (éch. $D_2O$, 2H, $NH_2$) ; 7,45 (m, 4H, aro.) ; 10,8 (éch. $D_2O$, 1H, NH).

Exemple 8

La (méthylthio-2 acétyl)-1 (orthochlorophényl-1 éthylidène-1)-2 hydrazine a été préparée à partir du méthylthioacétylhydrazide et de la chloro-2′ acétophénone, selon l'exemple 5.

RMN$^1$H DMSO$d_6$ : ppm: 2,15 (s, 3H, $CH_3$-S-) ; 2,25 (s, 3H, $CH_3$-C=N) ; 3,4 (s, 2H, $CH_2$) ; 7,45 (m, 4H, aro.) ; 10,5 (éch. $D_2O$, 1H, NH).

Exemple 9

Synthèse de l'oxamoyl-1 (orthochlorophényl-1 éthylidène-1)-2 hydrazine.

9.1. Dans un erlenmeyer muni d'un agitateur magnétique et d'un réfrigérant, on a introduit 25 ml d'éther éthylique et 0,1 mole d'oxamate d'éthyle. Au mélange maintenu à 0°C, on a ajouté goutte à goutte, sous agitation, 0,105 mole d'hydrate d'hydrazine. On a maintenu ensuite 2 h sous agitation à température ambiante. On a filtré et lavé le précipité d'hydrazide à l'éthanol froid puis à l'éther.

9.2. L'hydrazide obtenu (0,05 mole) a ensuite été solubilisé dans 50 ml d'éthanol-eau (50/50) et condensé avec la chloro-2′ acétophénone, comme indiqué dans l'exemple 1.

Rdt % = 82

PF (éthanol) = 175°C

RMN$^1$H DMSO$d_6$ : ppm : 2,25 (s, 3H, $CH_3$-S) ; 7,5 (m, 4H, aro.) ; 7,8 (éch. $D_2O$, 2H, $NH_2$) ; 10,6 (éch. $D_2O$, 1H, NH).

Exemple 10

Synthèse de l'(oxo-2 pyrrolidinyl-5)-1 (orthochlorophényl-1 éthylidène-1)-2 hydrazine.

10.1. L'(oxo-2 pyrrolidinyl-5) hydrazide a été préparé à partir de l'oxo-2 pyrrolidinyl-5 carboxylate d'éthyle par réaction avec l'hydrate d'hydrazine selon l'exemple 5.1.

10.2. L'hydrazide obtenu a été condensé avec la chloro-2′ acétophénone selon l'exemple 1.

Rdt % = 65

PF (éthanol-eau)
(base monohydratée)
RMN¹H $DMSOd_6$ : ppm : 2,05 (m, 2H, $\underline{CH_2}-\underset{\overset{\|}{O}}{C}$ ; 2,25 (s, 3H, $CH_3-C=N$) ; 3,30 (m, 2H, $-\underline{CH_2}-CH$) ; 4,5 (m, 1H, $\underline{CH}-\underset{\overset{\|}{O}}{C}$) ; 7,45 (m, 4H, aro.) ; 7,8 (éch. $D_2O$, 1H, $NH-\underset{\overset{\|}{H}}{C}-$) ; 10,4 (éch. $D_2O$, 1H, $NH-N=$).

Exemple 11

La nicotinoyl-1 (orthochlorophényl-1 éthylidène-1)-2 hydrazine a été préparée à partir du nicotinoylhydrazide et de la chloro-2′ acétophénone, selon l'exemple 5.
Rdt % = 62
PF (éthanol) = 154°C
RMN¹H $DMSOd_6$ : ppm : 2,25 (s, 3H, $CH_3-C=N$) ; 7,50 (m, 5H, aro.) ; 8,20 (m, 1H, aro.) ; 8,70 (m, 1H, aro.) ; 9,0 (m, 1H, aro.) ; 10,4 (éch. $D_2O$, 1H, NH).

Exemple 12

La formyl-1 (orthochlorophényl-1 éthylidène-1)-2 hydrazine a été préparée à partir du formylhydrazide et de la chloro-2′ acétophénone selon l'exemple 1.
Rdt % = 79
PF = 138°C (éthanol)
RMN¹ $DMSOd_6$ : ppm : 2,25 (s, 3H, $CH_3-C=N$) ; 7,45 (m, 4H, aro.) ; 8,8 (s, 1H, $H-\underset{\overset{\|}{O}}{C}$ ) ; 10,2 (éch. $D_2O$, 1H, NH).

Exemple 13

L'(amino-2 acétyl)-1 (parafluorophényl-1 éthylidène-1)-2 hydrazine a été préparée à partir du glycinoylhydrazide (chlorhydrate) et de la fluoro-4′acétophénone, selon l'exemple 7.
Rdt % = 72
PF (chlorhydrate) (méthanol) = 260°C
PF (base) (éthanol) = 139°C
RMN¹H $DMSOd_6$ : ppm : Chlorhydrate : 2,25 (s, 3H, $CH_3-C=N$) ; 3,8 (s, 2H, $CH_2$) ; 7,3 (m, 2H, aro.) ; 7,9 (m, 2H, aro.) ; 8,8 (éch. $D_2O$ ; 2H, $NH_2$) ; 10,4 (éch. $D_2O$, 1H, NH).
Base : 2,25 (s, 3H, $CH_3-C=N$) ; 3,6 (s, 2H, $CH_2$) ; 4,6 (éch. $D_2O$ ; 2H, $NH_2$) ; 7,3 (m, 2H, aro.) ; 7,9 (m, 2H, aro.) ; 10,3 (éch. $D_2O$, 1H, NH).

Exemple 14

L'(hydroxy-2 isobutyroyl)-1 (métatrifluorométhylphényl-1 éthylidène-1)-2 hydrazine a été préparée à partir de l'hydroxy-2 isobutyroyl-1 hydrazide et de la métatrifluoroacétophénone comme il est indiqué à l'exemple 1.
RMN¹H $CDCl_3$ : ppm : 1,54 (s, 6H, $2CH_3$) ; 2,24 (s, 3H, $CH_3-C=N$) ; 3,73 (éch. $D_2O$, 1H, OH) ; 7,40 (m, 4H, aro.) ; 8,00 (éch. $D_2O$, 1H, NH).

Exemple 15

La morpholinoacétyl-1 (orthochlorophényl-1 éthylidène-1)-2 hydrazine a été préparée à partir du morpholinoacétyl hydrazide et de la chloro-2′ acétophénone comme il est indiqué à l'exemple 5.
RMN¹H $CDCl_3$ : ppm : 2,42 (s, 3H, $CH_3$) ; 2,40 (t, 4H,2 $NCH_2$) ; 3,08 (s, 2H, $CH_2-C=O$) ; 3,43 (t, 4H,2 $OCH_2$ ; 3,75 (éch. $D_2O$, 1H, OH) ; 7,40 (m, 4H, aro.) ; 7,75 (éch. $D_2O$, 1H, NH).

Exemple 16

L'(hydroxy-2 isobutyroyl)-1 (orthochlorophényl-1 propylidène-1)-2 hydrazine a été préparée à partir de l'(hydroxy-2 isobutyroyl)-1 hydrazide et de l'orthochloropropiophénone comme il est indiqué à l'exemple 1.
RMN¹H $DMSOd_6$ : ppm : 1,25 (t, 3H, $\underline{CH_3}-CH_2$) ; 1,4 (s, 6H, $2CH_3$) ; 2,95 (q, 2H, $CH_2-\underset{\overset{\|}{N}}{C}-$) ; 5,8 (éch. $D_2O$, 1H, OH) : 7,5 (m, 4H, aro.) ; 10,2 (éch. $D_2O$, 1H, NH).

Exemple 17

L'(hydroxy-2 acétyl)-1 (orthofluorophényl-1 éthylidène-1)-2 hydrazine a été préparée selon l'exemple 1 à partir de l'hydroxy-2 acétyl hydrazide et de la fluoro-2′ acétophénone.
RMN¹H $DMSOd_6$ : ppm : 2,25 (s, 3H, $CH_3-C=N$) ; 4,2 (s, 2H, $CH_2$) ; 5,4 (éch. $D_2O$, 1H, OH) ; 7,8 (m, 4H, aro.) ; 10,6 (éch. $D_2O$, 1H, NH).

Exemple 18

L'(hydroxy-2 isobutyroyl)-1 (métachlorophényl-1 propylidène-1)-2 hydrazine a été préparée à partir de l'(hydroxy-2 isobutyroyl)-1 hydrazide et de la métachloropropiophénone, comme il est indiqué à l'exemple 1.

RMN[1]H DMSOd$_6$ : ppm : 1,25 (t, 3H, $CH_3$-$CH_2$) ; 1,4 (s, 6H, 2$CH_3$) ; 2,9 (q, 2H, $CH_2$- $\underset{\underset{N}{\|}}{C}$ ) ; 5,8 (éch. $D_2O$, 1H, OH) ; 7,40 (m, 2H, aro.) ; 7,80 (m, 2H, aro.) ; 9,5 (éch. $D_2O$, 1H, NH).
Rdt % = 68
PF (éthanol) = 112°C

Exemple 19

Synthèse de la N-acétylglycyl-1 (orthochlorophényl-1 éthylidène-1)-2 hydrazine.

Dans un erlenmeyer muni d'un système d'agitation magnétique et surmonté d'un réfrigérant sont introduits successivement 20 ml d'éthanol, 2 ml (0,04 mole) d'hydrate d'hydrazine et 5,80 g (0,04 mole) de N-acétylglycinate d'éthyle. Le mélange est chauffé pendant 2 h à 60°C puis on ajoute (sans refroidir) 6,18 g (0,04 mole) d'orthochloroacétophénone. L'agitation est maintenue pendant 2 h à 60°C. Après refroidissement, la solution est concentrée à l'évaporateur rotatif puis filtrée. Le solide obtenu est recristallisé dans l'isopropanol.
Rdt % = 83
PF = 188-190°C
RMN[1]H $CDCl_3$ : ppm : 2,32 (s, 3H, $CH_3$-C=N) ; 2,48 (s, 3H, $CH_3$-C=O) ; 4,60 (d, 2H, $CH_2$) ; 6,50 (s large, éch. $D_2O$, 2H, 2 x NH) ; 7,50 (s, 4H, aro.).

Exemple 20

L'(hydroxy-2 isobutyroyl)-1 (orthofluorophényl-1 éthylidène-1)-2 hydrazine a été préparée à partir de l'(hydroxy-2 isobutyroyl)-1 hydrazide et de l'orthofluoroacétophénone, comme il est indiqué à l'exemple 1.
Rdt % = 71
PF (éthanol) = 142°C
RMN[1]H DMSOd$_6$ : ppm : 1,40 (s, 6H, 2$CH_3$) ; 2,25 (s, 3H, $CH_3$-C=N) ; 5,8 (éch. $D_2O$, 1H, OH) ; 7,8 (m, 4H, aro.) ; 10,6 (éch. $D_2O$, 1H, HN).

Exemple 21

L'(hydroxy-2 isobutyroyl)-1 (parafluorophényl-1 éthylidène-1)-2 hydrazine a été préparée à partir de l'(hydroxy-2 isobutyroyl)-1 hydrazide et de la parafluoroacétophénone, comme il est indiqué à l'exemple 1.
Rdt % = 58
PF (éthanol) = 134°C
RMN[1]H DMSOd$_6$ : ppm : 1,40 (s, 3H, 2$CH_3$) ; 2,25 (s, 3H, $CH_3$-C=N) ; 5,6 (éch. $D_2O$, 1H, OH) ; 7,1 (m, 2H, aro.) ; 8,1 (m, 2H, aro.) ; 10,8 (éch. $D_2O$, 1H, NH).

Exemple 22

L'(hydroxy-2 isobutyroyl)-1 (orthobromophényl-1 éthylidène-1)-2 hydrazine a été préparée à partir de l'(hydroxy-2 isobutyroyl)-1 hydrazide et de l'orthobromoacétophénone, comme il est indiqué à l'exemple 1.
Rdt % = 69
PF (éthanol) = 139°C
RMN[1]H DMSOd$_6$ : ppm : 1,40 (s, 3H, 2$CH_3$) ; 2,25 (s, 3H, $CH_3$-C=N) ; 5,8 (éch. $D_2O$, 1H, OH) ; 7,6 (m, 4H, aro.) ; 10,6 (éch. $D_2O$, 1H, NH).

Exemple 23

L'(hydroxy-2 propanoyl)-1 (orthobromophényl-1 éthylidène-1)-2 hydrazine a été préparée à partir de l'(hydroxy-2 propanoyl)-1 hydrazide et de la bromo-2'acétophénone, comme il est indiqué à l'exemple 3.
Rdt % = 62
PF (éthanol) = 115°C
RMN[1]H DMSOd$_6$ : ppm : 1,3 (d, 3H, $CH_3$) ; 2,25 (s, 3H, $CH_3$-C=N) ; 4,2 (m, 1H, CH-OH) ; 5,6 (éch. $D_2O$, 1H, OH) ; 7,6 (m, 4H, aro.) ; 10,1 (éch. $D_2O$), 1H, NH).

Exemple 24

L'(hydroxy-2 acétyl)-1 (orthobromophényl-1 éthylidène-1)-2 hydrazine a été préparée à partir de l'(hydroxy-2 acétyl) hydrazide et de la bromo-2' acétophénone, comme il est indiqué à l'exemple 1.
Rdt % = 70
PF (éthanol) = 160°C
RMN[1]H DMSOd$_6$ : ppm : 2,25 (s, 3H, $CH_3$-C=N) ; 4,2 (s, 2H, $CH_2$) ; 5,4 (éch. $D_2O$, 1H, OH) ; 7,6 (m, 4H, aro.) ; 10,4 (éch. $D_2O$, 1H, NH).

Exemple 25

L'(hydroxy-2 isobutyroyl)-1 (paratrifluorométhylphényl-1 éthylidène-1)-2 hydrazine a été préparée à partir de l'hydroxy-2 isobutyroyl-1 hydrazide et de la métatrifluoroacétophénone, comme il est indiqué à l'exemple 1.
Rdt % = 69

PF (acétate d'éthyle) = 160°C
RMN$^1$H $CDCl_3$ : ppm : 1,57 (s, 6H, $H_3C$-C-OH) ; 2,32 (s, 3H, $H_3C$-C=O) ; 3,05 (éch. $D_2O$, 1H, OH) ; 7,30 (éch. $D_2O$, 1H, NH) ; 7,65 (d, 2H), J=10Hz, Aro.) ; 7,95 (d, 2H, J=10Hz, Aro.).

Exemple 26

L'(hydroxy-2 isobutyroyl)-1 (chloro-2 phényl-1 pentylidène-1)-2 hydrazine se prépare à partir de la (chloro-2 phényl)-1 pentanone-1. La (chloro-2 phényl)-1 pentanone-1 est obtenue en deux étapes à partir du chloro-2 benzaldéhyde : dans un premier temps, on ajoute 0,5 mole de chloro-2 benzaldéhyde dissous dans 100 ml d'éther anhydre à une suspension de bromure de n-butylmagnésium dans l'éther anhydre (obtenue par réaction de 0,5 mole de bromure de butyle avec 13 g de magnésium). Après hydrolyse du milieu réactionnel par 300 ml d'acide chlorhydrique 1 N, l'alcool est isolé par concentration de la phase organique puis distillation.
Rdt % = 78
$E_{0,1}$ = 114-118°C

L'alcool est dans un deuxième temps oxydé en (chloro-2 phényl)-1 pentanone-1 par l'acide chromique : un mélange de 0,5 mole de (chloro-2 phényl)-1 pentanol-1 et de 80 g de bichromate de sodium en solution dans 100 ml d'acétone est refroidi à 10°C. Au mélange on ajoute, sans que la température de la réaction dépasse 20°C, une solution de 50 ml d'acide sulfurique concentré dans 100 ml d'eau. Le mélange est ensuite agité 12 heures à température ambiante puis dilué dans 1 000 ml d'eau et extrait par l'éther. La solution organique est lavée jusqu'à neutralité par une solution aqueuse de bicarbonate de sodium puis par l'eau. Après concentration de la phase éthérée, la (chloro-2 phényl)-1 pentanone-1 est distillée.
Rdt % = 91
$E_{0,5}$ = 114-117°C

L'(hydroxy-2 isobutyroyl)-1 (chloro-2 phényl-1 pentylidène-1)-2 hydrazine est préparée par condensation de la (chloro-2 phényl)-1 pentanone-1 avec l'(hydroxy-2 isobutyroyl)-1 hydrazine selon l'exemple 1.
Rdt % = 78
PF (acétate d'éthyle) = 199°C
RMN$^1$H $CDCl_3$ : ppm : 0,95 (m, 3H, $H_3C$-$CH_2$) ; 1,46 (s, 6H, 2$CH_3$) ; 1,65 (m, 4H, $CH_2$-$CH_2$-$CH_3$) ; 2,37 (éch. $D_2O$, s, 1H, OH) ; 2,65 (t, 2H, $CH_2$C=N) ; 7,10-7,30 (m, 4H, aro.) ; 8,20 (s, 1H, NH).

Exemple 27

L'(hydroxy-2 propanoyl)-1 (orthofluorophényl-1 éthylidène-1)-2 hydrazine a été préparée à partir d'hydroxy-2 propanoylhydrazine et de la fluoro-2'acétophénone, selon les modalités de l'exemple 3.
Rdt % = 52
PF = 109°C (éthanol)
RMN$^1$H DMSO$d_6$ : ppm : 1,3 (d, 3H, $CH_3$) ; 2,25 (s, 3H, $CH_3$-C=N) ; 4,2 (m, 1H, CH-OH) ; 5,6 (éch. $D_2O$, 1H, OH) ; 7,8 (m, 4H, aro.) ; 10,8 (éch. $D_2O$, 1H, NH).

Exemple 28

L'(hydroxy-2 isobutyroyl)-1 (métachlorophényl-1 éthylidène-1)-2 hydrazine a été préparée à partir d'hydroxy-2 isobutyroylhydrazine et de la métachloroacétophénone, selon les modalités de l'exemple 1.
Rdt % = 68
PF = 136°C (éthanol)
RMN$^1$H DMSO$d_6$ : ppm : 1,4 (s, 6H, 2$CH_3$) ; 2,25 (s, 3H, $CH_3$-C=N) ; 5,9 (éch. $D_2O$, 1H, OH) ; 7,4 (m, 2H, aro.) ; 7,75 (m, 2H, aro.) ; 10,5 (éch. $D_2O$, 1H, NH).

Exemple 29

L'(hydroxy-2 propanoyl)-1 (métacholorophényl-1 éthylidène-1)-2 hydrazine a été préparée à partir d'hydroxy-2 propanoyl-hydrazine et de la métachloroacétophénone, selon les modalités de l'exemple 3.
Rdt % = 61
PF = 114°C (éthanol)
RMN$^1$H DMSO$d_6$ : ppm : 1,3 (d, 3H, $CH_3$) ; 2,25 (s, 3H, $CH_3$-C=N) ; 4,2 (m, 1H, CH-OH) ; 5,8 (éch. $D_2O$, 1H, OH) ; 7,45 (m, 2H, aro.) ; 7,7 (m, 2H, aro.) ; 11,2 (éch. $D_2O$, 1H, NH).

Exemple 30

L'(hydroxy-2 isobutyroyl)-1 (orthométhylphényl-1 éthylidène-1)-2 hydrazine a été préparée à partir d'hydroxy-2 isobutyroylhydrazine et de l'orthométhylacétophénone, selon les modalités de l'exemple 1.
Rdt % = 65
PF = 103°C (éthanol)
RMN$^1$H DMSO$d_6$ : ppm : 1,4 (s, 6H, 2$CH_3$) ; 2,25 (s, 3H, $CH_3$-C=N) ; 4,61 (s, 3H, C 1,4 (s, 6H, 2$CH_3$) ; 2,25 (s, 3H, $CH_3$-C=N) ; 4,61 (s, 3H, $CH_3$-aro.) ; 5,8 (éch. $D_2O$, 1H, OH) ; 7,6 à 8,3 (m, 4H, aro.) ; 11,2 (éch. $D_2O$, 1H, NH).

Exemple 31

La préparation de la S-(hydroxy-3 amino-2 propanoyl)-1 (chloro-2 phényl-1 éthylidène-1)-2 hydrazine comporte les stades suivants.

A 0,3 mole de chlorhydrate de l'ester éthylique de la L-sérine en suspension dans 100 ml d'acétate d'éthyle, on ajoute 0,4 mole de triéthylamine. Le mélange est agité 15 minutes à température ambiante puis filtré. Le précipité de chlorhydrate de triéthylamine est lavé deux fois avec 10 ml d'acétate d'éthyle. La solution organique est évaporée sous vide. On obtient l'ester éthylique de la L-sérine sous forme d'une huile. Cette huile est dissoute dans 50 ml de méthanol puis on ajoute 0,35 mole d'hydrate d'hydrazine. Le mélange est agité 12 heures à température ambiante puis concentré sous vide. L'hydrazide de la sérine cristallise, il est lavé par 5 ml d'éthanol absolu et par deux fois 10 ml d'éther. Rdt % = 64

L'(hydroxy-3 amino-2 propanoyl)-1 (chloro-2 phényl)-1 éthylidène)-2 hydrazine est obtenue par condensation de l'hydrazide de la L-sérine avec la chloro-2′ acétophénone selon l'exemple 1.

Rdt % = 71
PF (acétate d'éthyle) = 120-123°C
RMN$^1$H $CDCl_3$ : ppm : 2,35 (s, 3H, $CH_3$) ; 3,80 (m, 3H, $CHCH_2$) ; 4,20 (éch. $D_2O$, s, 4H, $NH+NH_2+OH$) ; 7,25 (s, 4H, aro.).

Exemple 32

Le chlorhydrate de l'aminoacétyl-1 (paraméthylphényl-1 éthylidène-1)-2 hydrazine a été préparée à partir du chlorhydrate de l'aminoacétylhydrazine et de la paraméthylacétophénone, selon les modalités de l'exemple 7.

Rdt % = 58
PF = 260°C (éthanol)
RMN$^1$H DMSO$d_6$ : ppm : 2,25 (s, 3H, $CH_3$-C=N) ; 2,5 (s, 3H, $CH_3$-aro.); 3,8 (s, 2H, $CH_2$) ; 7,1 (m, 2H, aro.) ; 7,7 (m, 2H, aro.) ; 8,8 (éch. $D_2O$, 3H, $NH_3$ $^+$) ; 10,8 (éch. $D_2O$, 1H, NH).

Exemple 33

La salicoyloxy-1 (orthochlorophényl-1 éthylidène-1)-2 hydrazine a été préparée à partir de la salicoylhydrazine et de la chloro-2′ acétophénone, selon les modalités de l'exemple 1.

Rdt % = 71
PF = 216°C (éthanol)
RMN$^1$H DMSO$d_6$ : ppm : 2,25 (s, 3H, $CH_3$-C=N) ; 7,1 (m, 5H, aro.) ; 7,8 (m, 4H, aro.) ; 8,4 (éch. $D_2O$, 1H, OH) ; 10,8 (éch. $D_2O$, 1H, NH).

Exemple 34

L'(hydroxy-2 isobutyroyl)-1 (métaméthylphényl-1 éthylidène-1)-2 hydrazine a été préparée à partir d'hydroxy-2 isobutyroylhydrazine et de la métaméthylacétophénone, selon les modalités de l'exemple 1.

Rdt % = 59
PF = 126°C (éthanol)
Rdt % = 59
PF = 126°C (éthanol)
RMN$^1$H DMSO$d_6$ : ppm : 1,4 (s, 6H, $2CH_3$) ; 2,25 (s, 3H, $CH_3$-C=N) ; 3,6 (s, 3H, $CH_3$) ; 5,9 (éch. $D_2O$, 1H, OH) ; 6,9 à 7,8 (m, 4H, aro.) ; 10,8 (éch. $D_2O$, 1H, NH).

Exemple 35

La (pipéridino-glyoxyl)-1 (orthochlorophényl-1 éthylidène-1)-2 hydrazine a été préparée à partir de la pipéridinoglyoxylhydrazine et de l'orthochloroacétophénone selon les modalités de l'exemple 1.

Rdt % = 73
PF = 141°C (éthanol)
RMN$^1$H DMSO$d_6$ : ppm : 1,65 (m, 6H, $3CH_2$) ; 2,25 (s, 3H, $CH_3$-C=N) ; 3,4 (m, 4H, $2CH_2$) ; 7,5 (m, 5H, aro.) ; 10,9 (éch. $D_2O$, 1H, NH).

Exemple 36

Préparation de la S-(N-benzoylséryl)-1 (chloro-2 phényl-1 éthylidène-1)-2 hydrazine.

L'ester éthylique de la N-benzoyl sérine est obtenu à partir du chlorhydrate de l'ester éthylique de la L-sérine : le chlorhydrate (0,4 mole en suspension dans 100 ml d'acétate d'éthyle) est additionné d'une mole de triéthylamine. Le mélange est refroidi à 0°C puis on ajoute 0,4 mole de chlorure de benzoyle en solution dans 100 ml d'acétate d'éthyle. Après la fin de l'addition, le mélange est porté 15 minutes à 40°C. Le mélange est filtré, le précipité lavé par deux fois 50 ml d'acétate d'éthyle. Le filtrat, évaporé sous vide, laisse l'ester éthylique de la N-benzoylsérine sous forme d'une huile qui est dissoute dans 60 ml de méthanol et additionnée de 0,45 mole d'hydrate d'hydrazine. Après 12 heures d'agitation à température ambiante, le mélange est concentré sous vide. L'hydrazide qui a cristallisé est lavé par 10 ml de méthanol puis par deux fois 10 ml d'éther éthylique.

Rdt % = 84

L'hydrazide de la N-benzoylsérine est condensé avec la (chloro-2 phényl)-1 éthanone comme il est indiqué dans l'exemple 1.

Rdt % = 88
PF (éthanol) = 183-187°C

RMN$^1$H DMSO$d_6$ : ppm : 2,37 (s, 3H, $CH_3$) ; 3,92 (m, 3H, CH-$CH_2$OH) ; 5,25 (éch. $D_2O$, m, 2H, OH+NHCO) ; 7,25-8,15 (m, 9H, aro.) ; 8,75 (éch. $D_2O$, s, 1H, N-NH).

L'étude toxico-pharmacologique des produits de l'invention fait apparaître les résultats suivants : pour l'(hydroxy-2 isobutyroyl)-1 (orthochlorophényl-1 éthylidène-1)-2 hydrazine (MP 440) :

| | D.L. 50 (SOURIS) | D.L.(50 RAT) |
|---|---|---|
| I.P. | = 172 $mg.kg^{-1}$ (0,67 mmol) | |
| P.O. | = 347 $mg.kg^{-1}$ (1,36 mmol) | P.O. = 970 $mg.kg^{-1}$ (3,8 mmol) |

A titre de comparaison, on indiquera ci-après la toxicité de deux composés analogues dichlorés :

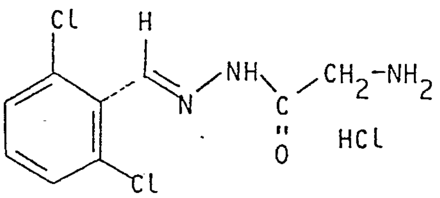

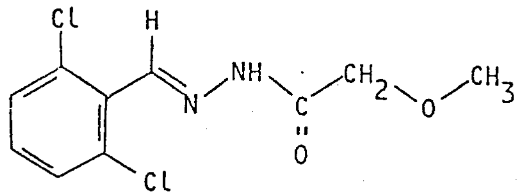

$DL_{50}IP$ = 71 mg/kg
$DL_{50}PO$ = 114 mg/kg

$DL_{50}IP$ = 105 mg/kg
$DL_{50}PO$ = 138 mg/kg

L'activité antihypertensive de ces deux composés respectivement se manifeste aux doses de :
6,25 mg/kg per os et 1,56 mg/kg per os
(soit 1/18$^e$ de la $DL_{50}$) (1/88$^e$ de la $DL_{50}$)

Par contre, l'activité antihypertensive chez le rat SHR du composé de l'invention ci-dessus est de 2 mg/kg per os soit 1/485° de la $DL_{50}$.

On note donc que les composés de l'invention ont une toxicité bien moindre que les composés dichlorés analogues.

PHARMACOLOGIE

a) Chez la souris

La molécule de l'(hydroxy-2-isobutyroyl)-1 (orthochlorophényl-1 éthylidène-1)-2 hydrazine :
- diminue la motilité spontanée de la souris ($D.E._{50}$ = 10 $mg.kg^{-1}$ I.P.)
- antagonise l'hypermotilité amphétaminique à partir de 12,5 $mg.kg^{-1}$ I.P.
- diminue l'activité d'exploration ($D.E._{50}$ = 27 $mg.kg^{-1}$ I.P.)
- entraîne une incoordination motrice et une hypothermie uniquement à forte dose (50 $mg.kg^{-1}$ I.P.)
- présente une activité analgésique :
. faible au test à la P.B.Q.
. plus nette avec la "plaque chauffante"
($D.E._{50}$ = 6 $mg.kg^{-1}$ I.P.)
- potentialise le sommeil barbiturique dès la dose de 12,5 $mg.kg^{-1}$ I.P.

Le produit est inactif sur les tests de la yohimbine et à l'apomorphine et modifie de façon peu nette le test à l'oxotrémorine à forte dose.

b) Chez le rat

L'(hydroxy-2 isobutyroyl)-1 (orthochlorophényl-1 éthylidène-1)-2 hydrazine se révèle anti-inflammatoire sur le test à la carraghénine ($D.E._{50}$ = 38 $mg.kg^{-1}$ I.P.)

Sur le plan cardio-vasculaire, la molécule :

- augmente significativement le rythme cardiaque du rat normotendu, sans modifier la pression artérielle à 50 $mg.kg^{-1}$ I.P.

- diminue de façon importante la pression artérielle du rat spontanément hypertendu (S.H.R.) en administration unique dès les doses très inférieures aux doses sédatives et toxiques ($D.E._{50}$ = 1,9 $mg.kg^{-1}$

I.P.).

Les essais en administration P.O. confirment l'action antihypertensive du produit :

- en administration unique (D.E.$_{50}$ = 2 mg.kg$^{-1}$)
- en administration répétée sur 5 jours, à la dose de 1,5 mg.kg$^{-1}$.

La fréquence cardiaque chez l'animal n'est pas modifiée de manière significative dans les conditions expérimentales définies.

Le profil pharmacologique de l'(hydroxy-2 isobutyroyl)-1 (orthochlorophényl-1 éthylidène-1)-2 hydrazine est celui d'une substance antihypertensive qui présente également des propriétés-sédatives, anti-inflammatoires et analgésiques, mais à des doses supérieures à celles requises pour l'activité antihypertensive.

L'activité antihypertensive (SHR) d'autres composés de l'invention a également été déterminée et comparée à celle de composés de l'art antérieur ($R_1$ = H) ou à celle de composés alkylés ($R_1$ = alkyle) qui se distinguent des composés de l'invention par leurs caractéristiques structurales (X et $R_2$).

Dans le tableau II ci-après, les composés de l'invention sont identifiés par le numéro de leur exemple de préparation et suivis chacun par le ou les composés utilisés à titre de comparaison, lesquels portent le même numéro affecté d'un indice a, b... Les résultats obtenus, qui figurent dans le tableau II, montrent que seuls les composés de l'invention présentent une activité antihypertensive.

TABLEAU II

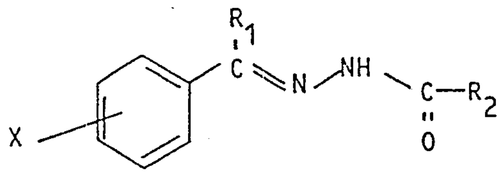

IV

| Composé de l'exemple n° | X | $R_1$ | $R_2$ | Activité anti-hypertensive SHR Dose minimale active | DL 50 (*) |
|---|---|---|---|---|---|
| 1 | o-Cl | $CH_3$ | $CH_3$<br>$-C-CH_3$<br>OH | 2 $mg.kg^{-1}$ P.O. | 482 $mg.kg^{-1}$ I.P. |
| 1a | o-Cl | H | $CH_3$<br>$-C-CH_3$<br>OH | inactif | 490 $mg.kg^{-1}$ I.P. |
| 1b | p-Cl | $CH_3$ | $CH_3$<br>$-C-CH_3$<br>OH | inactif | 150 $mg.kg^{-1}$ I.P. |
| 1c | p-Cl | H | $CH_3$<br>$-C-CH_3$<br>OH | inactif | 660 $mg.kg^{-1}$ I.P. |
| 1d | o-Cl | H | $CH_3$<br>$-CH_2-C-CH_3$<br>OH | inactif | 433 $mg.kg^{-1}$ I.P. |
| 1e | o-Cl | $CH_3$ | $CH_3$<br>$-CH_2-C-CH_3$<br>OH | inactif | 227 $mg.kg^{-1}$ I.P. |
| 3 | o-Cl | $CH_3$ | $-CHOHCH_3$ | 12,5 $mg.kg^{-1}$I.P. | 240 $mg.kg^{-1}$ I.P. |
| 3a | o-Cl | H | $-CHOHCH_3$ | inactif | 300 $mg.kg^{-1}$ |
| 4 | o-Cl | $CH_3$ | $-CH_2OH$ | 3,13 $mg.kg^{-1}$ | 583 $mg.kg^{-1}$ I.P. |

(*) I.P. = voie intrapéritonéale
P.O. = voie orale

TABLEAU II (Suite)

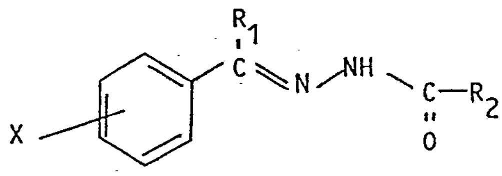

IV

| Composé de l'exemple n° | X | $R_1$ | $R_2$ | Activité anti-hypertensive SHR Dose minimale active | DL 50 (*) |
|---|---|---|---|---|---|
| 6<br>6a | p-Cl<br>p-Cl | $CH_3$<br>H | $-CH_2OCH_3$<br>$-CH_2OCH_3$ | 6,25 $mg.kg^{-1}$ I.P.<br>inactif | 200 $mg.kg^{-1}$ I.P.<br>477 $mg.kg^{-1}$ I.P. |
| 7<br>7a | p-Cl<br>p-Cl | $CH_3$<br>H | $-CH_2-NH_2-HCl$<br>$-CH_2-NH_2-HCl$ | 2 $mg.kg^{-1}$ P.O.<br>inactif | 143 $mg.kg^{-1}$ I.P.<br>360 $mg.kg^{-1}$ I.P. |
| 8 | p-Cl | $CH_3$ | $-CH_2-S-CH_3$ | 12,5 $mg.kg^{-1}$ I.P. | >400 $mg.kg^{-1}$ I.P. |
| 9<br>9a | p-Cl<br>p-Cl | $CH_3$<br>H | $-CO-NH_2$<br>$-CO-NH_2$ | 12,5 $mg.kg^{-1}$ I.P.<br>inactif | >400 $mg.kg^{-1}$ I.P.<br>>400 $mg.kg^{-1}$ I.P. |
| 16 | p-Cl | $C_2H_5$ | $-\overset{CH_3}{\underset{OH}{C}}-CH_3$ | 6,25 $mg.kg^{-1}$ I.P. | 179 $mg.kg^{-1}$ I.P. |
| 26 | p-Cl | $C_4H_9$ | $-\overset{CH_3}{\underset{OH}{C}}-CH_3$ | 6,25 $mg.kg^{-1}$ I.P. | >400 $mg.kg^{-1}$ I.P. |

(*) I.P. = voie intrapéritonéale
P.O. = voie orale

## Revendications

1. Dérivés d'hydrazine répondant à la formule

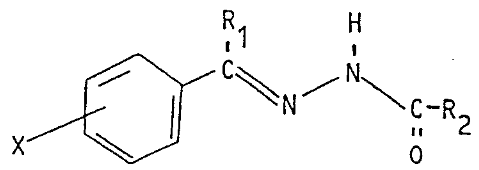

dans laquelle
X est un alkyle en $C_1$-$C_4$, le groupe trifluorométhyle ou un halogène choisi parmi le chlore (en position ortho ou méta) le fluor ou le brome,
$R_1$ est un groupe alkyle en $C_1$-$C_4$ ;
$R_2$ représente :
- l'hydrogène ;
- un groupe alkyle ramifié en $C_3$-$C_4$ ;
- un groupe alcoxy en $C_1$-$C_4$, de préférence éthoxy ;
- un groupe de formule $CR_3R_4R_5$ dans lequel :
. $R_3$ représente un groupe en $OR_6$, $SR_6$ ou $NR_7R_8$
dans lesquels:
$R_6$ est l'hydrogène ou un alkyle $C_1$-$C_4$ ;
$R_7$ et $R_8$ représentent l'hydrogène, un groupe alkyle en $C_1$-$C_4$, un groupe acyle en $C_2$-$C_4$, un groupe benzoyle ou bien $R_7$ et $R_8$ ensemble avec l'atome d'azote auquel ils sont liés forment un groupe hétérocyclique à 5 ou 6 chaînons ;
. $R_4$ et $R_5$ sont identiques et représentent l'hydrogène,ou un groupe alkyle en $C_1$-$C_4$ ou bien $R_4$ est l'hydrogène et $R_5$ représente soit un groupe alkyle en $C_1$ - $C_4$ éventuellement substitué par un groupe hydroxy, soit un groupe phényle ; ou bien
. $R_3$, $R_4$, et $R_5$, ensemble avec l'atome de carbone auquel ils sont liés, forment un groupe amido ;
- un groupe phényle éventuellement substitué par un groupe hydroxy ;
- un groupe hétérocycle à 5 ou 6 chaînons et leurs sels pharmaceutiquement acceptables.

2 - Dérivés selon la revendication 1, caractérisés en ce qu'ils sont l'un des composés ci-après :
- (hydroxy-2 isobutyroyl)-1 (orthochlorophényl-1 éthylidène-1)-2 hydrazine,
- (triméthyl-2,2,2 acétyl)-1 (orthochlorophényl-1 éthylidène-1)-2 hydrazine,
- (hydroxy-2 propanoyl)-1 (orthochlorophényl-1 éthylidène-1)-2 hydrazine,
- (hydroxy-2 acétyl)-1 (orthochlorophényl-1 éthylidène-1)-2 hydrazine,
- (hydroxy-2 phényl-2 acétyl)-1 (orthochlorophényl-1 éthylidène-1)-2 hydrazine,
- (méthoxy-2 acétyl)-1 (orthochlorophényl-1 éthylidène-1)-2 hydrazine,
- (amino-2 acétyl)-1 (orthochlorophényl-1 éthylidène-1)-2 hydrazine,
- (méthylthio-2 acétyl)-1 (orthochlorophényl-1 éthylidène-1)-2 hydrazine,
- oxamoyl-1 (orthochlorophényl-1 éthylidène-1)-2 hydrazine,
- (oxo-2 pyrrolidinyl-5)-1 (orthochlorophényl-1 éthylidène-1)-2 hydrazine,
- nicotinoyl-1 (orthochlorophényl-1 éthylidène-1)-2 hydrazine,
- formyl-1 (orthochlorophényl-1 éthylidène-1)-2 hydrazine,
- (amino-2 acétyl)-1 (parafluorophényl-1 éthylidène-1)-2 hydrazine,
- (hydroxy-2 isobutyroyl)-1 (métatrifluorométhylphényl-1 éthylidène-1)-2 hydrazine,
- morpholinoacétyl-1 (orthochlorophényl-1 éthylidène-1)-2 hydrazine,
- (hydroxy-2 isobutyroyl)-1 (orthochlorophényl-1 propylidène-1)-2 hydrazine,
- (hydroxy-2 isobutyroyl)-1 (parachlorophényl-1 propylidène-1)-2 hydrazine,
- (hydroxy-2 acétyl)-1 (orthofluorophényl-1 éthylidène-1)-2 hydrazine,
- (hydroxy-2 isobutyroyl)-1 (métachlorophényl-1 propylidéne-1)-2 hydrazine,
- N-acétylglycyl-1 (orthochlorophényl-1 éthylidène-1)-2 hydrazine,
- (hydroxy-2 isobutyroyl)-1 (orthochlorophényl-1 éthylidène-1)-2 hydrazine,
- (hydroxy-2 isobutyroyl)-1 (parafluorophényl-1 éthylidène-1)-2 hydrazine,
- (hydroxy-2 isobutyroyl)-1 (orthobromophényl-1 éthylidène-1)-2 hydrazine,
- (hydroxy-2 propanoyl)-1 (orthochlorophényl-1 éthylidène-1)-2 hydrazine,
- (hydroxy-2 acétyl)-1 (orthobromophényl-1 éthylidène-1)-2 hydrazine,
- (hydroxy-2 isobutyroyl)-1(paratrifluorométhylphényl-1 éthylidène-1)-2 hydrazine,
- (hydroxy-2 isobutyroyl)-1 chloro-2 phényl-1 pentylidène-1)-2 hydrazine,

- (hydroxy-2 propanoyl)-1 (orthofluorophényl-1 éthylidène-1)-2 hydrazine,
- (hydroxy-2 isobutyroyl)-1 (métachlorophényl-1 éthylidène-1)-2 hydrazine,
- (hydroxy-2 propanoyl)-1 (métachlorophényl-1 éthylidène-1)-2 hydrazine,
- (hydroxy-2 isobutyroyl)-1 (orthométhylphényl-1 éthylidène-1)-2 hydrazine,
- S-(hydroxy-3 amino-2 propanoyl)-1 chloro-2 phényl-1 éthylidène-1)-2 hydrazine,
- chlorhydrate de l'aminoacétyl-1 (métaméthoxyphényl-1 éthylidène-1)-2 hydrazine,
- chlorhydrate de l'aminoacétyl-1 (paraméthylphényl-1 éthylidène-1)-2 hydrazine,
- salicyloyl-1 (orthochlorophényl-1 éthylidène-1)-2 hydrazine,
- (hydroxy-2 isobutyroyl)-1 (métaméthylphényl-1 éthylidène-1)-2 hydrazine,
- (pipéridino-glyoxyl)-1 orthochlorobenzylidène-2 hydrazine,
- S-(N-benzoylséryl)-1 (chloro-2 phényl-1 éthylidène-1)-2 hydra zine.

3 - Procédé de synthèse des dérivés selon la revendication 1, caractérisé en ce qu'il consiste :

1) à préparer, dans une première étape, une acylhydrazine de formule (1)

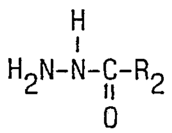

(1)

à partir d'hydrate d'hydrazine et d'un ester de formule (2) $R_2$-COOR dans laquelle $R_2$ est tel que défini dans la revendication 1,

2) puis à faire réagir dans une seconde étape le produit (1) avec une arylalkylcétone de formule (3)

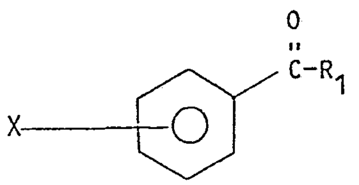

(3)

dans laquelle X et $R_1$ sont tel que définis dans la revendication 1 pour obtenir le dérivé d'hydrazine de formule IV.

4 - Compositions pharmaceutiques, caractérisées en ce qu'elles contiennent à titre de principe actif un dérivé d'hydrazine selon l'une des revendications 1 ou 2, en combinaison avec un véhicule pharmaceutiquement acceptable.

5 - Dérivés d'hydrazine selon la revendication 1, caractérisés en ce que $R_2$ est le groupe $-CR_3R_4R_5$, tel que défini dans la revendication 1.

6 - Dérivés selon l'une des revendications 1 ou 5, caractérisés en ce que X est un halogène choisi parmi le chlore (en ortho ou méta), le fluor ou le brome.

7 - Dérivés selon l'une quelconque des revendications 1, 5 ou 6 caractérisés en ce que $R_1$ est le groupe méthyle.

8 - Dérivé selon l'une quelconque des revendications 1, 5 à 6, caractérisé en ce que $R_1$ est le groupe méthyle, X est le chlore en ortho et $R_2$ est le groupe

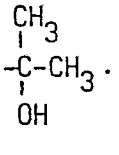

.

**Revendications pour les états contracants suivants: ES et GR.**

1 - Procédé pour l'obtention de dérivés d'hydrazine répondant à la formule

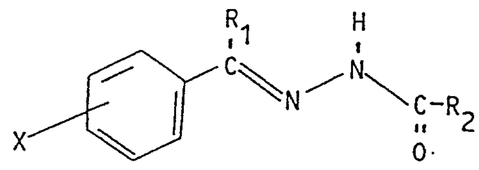

dans laquelle
X est un alkyle en $C_1$-$C_4$, le groupe trifluorométhyle ou un halogène choisi parmi le chlore (en position ortho ou méta) le fluor ou le brome,
$R_1$ est un groupe alkyle en $C_1$-$C_4$ ;
$R_2$ représente :
- l'hydrogène ;
- un groupe alkyle ramifié en $C_3$-$C_4$ .
- un groupe alcoxy en $C_1$-$C_4$, de préférence éthoxy ;
- un groupe de formule $CR_3R_4R_5$ dans lequel :
. $R_3$ représente un groupe en $OR_6$, $SR_6$ ou $NR_7R_8$
dans lesquels:
$R_6$ est l'hydrogène ou un alkyle $C_1$-$C_4$ .
$R_7$ et $R_8$ représentent l'hydrogène, un groupe alkyle en $C_1$-$C_4$, un groupe acyle en $C_2$-$C_4$, un groupe benzoyle ou bien $R_7$ et $R_8$ ensemble avec l'atome d'azote auquel ils sont liés forment un groupe hétérocyclique à 5 ou 6 chaînons ;
. $R_4$ et $R_5$ sont identiques et représentent l'hydrogène,ou un groupe alkyle en $C_1$-$C_4$ ou bien $R_4$ est l'hydrogène et $R_5$ représente soit un groupe alkyle en $C_1$ - $C_4$ éventuellement substitué par un groupe hydroxy, soit un groupe phényle ; ou bien
. $R_3$, $R_4$, et $R_5$, ensemble avec l'atome de carbone auquel ils sont liés, forment un groupe amido ;
- un groupe phényl éventuellement substitué par un groupe hydroxy ;
- un groupe hétérocyclique à 5 ou 6 chaînons et de leurs sels pharmaceutiquement acceptables, caractérisé en ce qu'il consiste :
1) à préparer, dans une première étape, une acylhydrazine de formule (1)

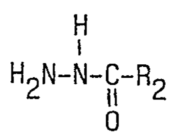

(1)

à partir d'hydrate d'hydrazine et d'un ester de formule (2) $R_2$-COOR dans laquelle $R_2$ est tel que défini dans la revendication 1,
2) puis à faire réagir dans une seconde étape le produit (1) avec une arylalkylcétone de formule (3)

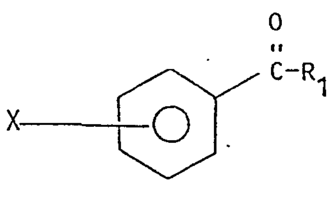

(3)

dans laquelle X et $R_1$ sont tels que définis dans la revendication 1 pour obtenir le dérivé d'hydrazine de formule IV et éventuellement à transformer le dérivé obtenu en ses sels pharmaceutiquement acceptables.

2 - Procédé selon la revendication 1, caractérisé en ce que les deux étapes sont réalisées au reflux dans un milieu alcoolique.

3 - Procédé selon l'une des revendications 1 ou 2, caractérisé en ce que le milieu alcoolique est l'éthanol ou un mélange éthanol/eau.

4 - Procédé selon l'une quelconque des revendications 1 à 3, caractérisé en ce que l'on utilise une acylhydrazine de formule (1) dans laquelle $R_2$ est un groupe de formule -$CR_3R_4R_5$ tel que défini dans la revendication 1.

5 - Procédé selon l'une quelconque des revendications 1 à 4, caractérisé en ce que l'on utilise une arylalkylcétone de formule (3) dans laquelle X est un halogène choisi parmi le chlore (en ortho ou méta) le

brome ou le fluor et $R_1$ est tel que défini dans la revendication 1.

6 - Procédé selon la revendication 5, caractérisé en ce que l'on utilise un arylalkylcétone de formule (3) dans laquelle X est un halogène tel que défini dans la revendication 1 et $R_1$ est le groupe méthyle.

7 - Procédé selon l'une quelconque des revendications 1 à 6, caractérisé en ce que l'on utilise une acylhydrazine de formule (1) dans laquelle $R_2$ est le groupe de formule

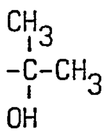

et une arylalkylcétone de formule (3) dans laquelle $R_1$ est $CH_3$ et X est le chlore en ortho.

Office européen des brevets

# RAPPORT DE RECHERCHE EUROPEENNE

Numero de la demande

EP 88 40 1149

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | J. AM. CHEM. SOC., vol. 75, 5 mai 1953, pages 2259-2261, Washington, D.C., US; N. RABJOHN et al.: "Carbazic acid esters as carbonyl reagents" * Page 2260, tableaux * | 1,3,7 | C 07 C 109/12<br>C 07 D 133/00<br>A 61 K 31/75<br>A 61 K 31/325<br>A 61 K 31/27 |
| X | J. HETEROCYCLIC CHEM. vol. 22, mars/avril 1985, pages 501-504, Provo, US; A.M. HUFF et al.: "The preparation of phenacylpyrazoles, acylpyrazotes, imidazolylisoxazoles and imidazolylpyrazoles from C(alpha)-dianions of oximes, phenylhydrazones and acylhydrazones" * Page 501, colonne de gauche, schéma; page 502, tableau, composés 2,3 * | 1,7 | |
| X | J. HETEROCYCLIC CHEM., vol. 24, mai/juin 1987, pages 555-559, Provo, US; D.C. DUNCAN et al.: "The preparation of N-carboalkoxypyrazoles and N-phenylpyrazoles from C(alpha)-dianions of carboalkoxyhydrazones and phenylhydrazones" * Pages 556-557, tableau; page 559, note 20 * | 1,3,7 | **DOMAINES TECHNIQUES RECHERCHES (Int. Cl.4)**<br>C 07 C 109/00<br>C 07 C 133/00<br>A 61 K 31/00 |
| X | CHEMICAL ABSTRACTS, vol. 95, no. 25, 21 décembre 1981, page 539, résumé no. 220282v, Columbus, Ohio, US; V.S. MISRA et al.: "Synthesis and amoebicidal activity of some new N-(2-nitro-4,5-dimethoxybenzoyl) glycine hydrazones", & J. INDIAN CHEM. SOC. 1981, 58(10), 1020-1 * En entier * | 1,3,7 | |

-/-

Le présent rapport a été établi pour toutes les revendications

| Lieu de la recherche | Date d'achèvement de la recherche | Examinateur |
|---|---|---|
| LA HAYE | 23-08-1988 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 88 40 1149

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 95, no. 5, 3 août 1981, page 711, résumé no. 42566f, Columbus, Ohio, US; Ya.V. ZACHINYAEV et al.: "Carbothoxyhydrazones", & KHIM. PROM-ST., SER.: REAKT. OSOBO CHIST. VESHCHESTVA 1980, (6), 26-8 * En entier * --- | 1,3,7 | |
| X | CHEMICAL ABSTRACTS, vol. 86, no. 21, 23 mai 1977, page 464, résumé no. 155559p, Columbus, Ohio, US; A.K. SRIVASTAVA et al.: "Studies on organic fluorine compounds. Systhesis of fluorinated hydrazones, aryl esters of p-halogenated phenols, fluorinated hydroxy ketones and their thiosemicarbazones and fluoro-substituted thiazoles and their mercury derivatives as possible fungicides", & J. INDIAN, CHEM. SOC. 1976, 53(8), 841-5 * En entier * --- | 1,3,7 | DOMAINES TECHNIQUES RECHERCHES (Int. Cl.4) |
| X | J. ORG. CHEM., vol. 41, no. 24, 1976, pages 3805-3811, Washington D.C., US; H.E. BAUMGARTEN et al.: "Reactions of amines. 20. Syntheses of racemic and optically active alkylhydrazines and N-acyl-N-alkyl- and N-acyl-N-arylhydrazines" * Page 3807, tableau 1 * --- | 1,3,7 | |
| X | DD-A- 107 255 (UHLIG) * Page 2, lignes 20,21 * --- -/- | 1,3,5,7 | |

Le présent rapport a été établi pour toutes les revendications

| Lieu de la recherche | Date d'achèvement de la recherche | Examinateur |
|---|---|---|
| LA HAYE | 23-08-1988 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 88 40 1149

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-3 867 425 (DUSZA) * Exemples 9,10; colonne 1, ligne 17 - colonne 2, ligne 7 * --- | 1,3,4,7 | |
| X | J. CHEM. SOC. PERKIN I, 1974, pages 30,31, Londres, GB; A. CAPLIN: "Synthesis and 1H nuclear magnetic resonance spectra of some aryl-1,2,3-selenadiazoles" * Pages 30,31 * --- | 1,3,5,7 | |
| X | CHEMICAL ABSTRACTS, vol. 78, no. 17, 30 avril 1973, page 454, résumé no. 110989x, Columbus, Ohio, US; D. NASIPURI et al.: "Diazoalkanes. III. Reactions of diazomethane with alpha-methoxymethylene ketones", & INDIAN J. CHEM. 1972, 10(9), 897-904 * En entier * & 9TH COLLECTIVE INDEX, page 18908CS --- | 1,3,5 | |
| | | | DOMAINES TECHNIQUES RECHERCHES (Int. Cl.4) |
| X | BULL. SOC. CHIM. FR., no. 10, 1970, pages 3609-3616, Paris, FR; G. TSATSAS et al.: "Méthode générale d'obtention des aryl-3-diméthyl-3,4-pentanolides-4 par transposition intramoléculaire" * Page 3612, tableau 1 * --- | 1,3,5 | |
| X | REC. TRAV. CHIM. PAYS-BAS, vol. 88, 1969, pages 52-61, La Haye, NL; H. VAN BEKKUM et al.: "Electronic and steric effects in the hydrogenation of alkyl aryl ketones on palladium" * Page 53: "Materials" * --- -/- | 1,3,5 | |

Le présent rapport a été établi pour toutes les revendications

| Lieu de la recherche | Date d'achèvement de la recherche | Examinateur |
|---|---|---|
| LA HAYE | 23-08-1988 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

Office européen des brevets

# RAPPORT DE RECHERCHE EUROPEENNE

Numero de la demande

EP 88 40 1149

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | LIEBIGS ANN. CHEM., vol. 717, 1968, pages 80-90, Weinheim, DE; B. EISTERT et al.: "Umsetzungen von o- und m-Trifluormethyl-benzaldehyd mit Diazomethan und -äthan" * Pages 87,89 * --- | 1,3,5,7 | |
| X | COMPTES RENDUS, ACAD. SC. PARIS, vol. 267, série C, 14 octobre 1968, pages 976-979, Paris, FR; P. GRAMMATICAKIS: "Remarques sur la préparation et l'absorption dans l'ultraviolet moyen des benzoyl- et phénylcarbamyl-hydrazones des acétophénones substituées" * Pages 976; page 977, figures II,III * --- | 1,3,7 | |
| X | J. PHARM. SC., vol. 57, no. 7, juillet 1968, pages 1255-1256, Washington D.C., US; V.A.E. VOYATZAKIS et al.: "Influence of metallic ions on the antituberculous activity of isonicotinoyl hydrazones" * Tableau I, composé A11 * --- | 1,3,5,7 | DOMAINES TECHNIQUES RECHERCHES (Int. Cl.4) |
| X | BULL. SOC. CHIM. FRANCE, no. 9, 1969, pages 3451-3457, Paris, FR; P. CAUBERE: "Etude de la réactivité des halobenzènes en présence d'hexaméthylphosphotriamide (HMPT)(II)" * Pages 3455-3456 * --- -/- | 1,3,7 | |

Le présent rapport a été établi pour toutes les revendications

| Lieu de la recherche | Date d'achèvement de la recherche | Examinateur |
|---|---|---|
| LA HAYE | 23-08-1988 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 88 40 1149

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | HELVETICA CHIMICA ACTA, vol. 50, no. 6, 1967, pages 1461-1469, Bâle, CH; Y.-R. NAVES et al.: "Sur les spectres d'absorption infrarouge, entre 2,5 et 16mum, des semicarbazones" * Page 1468 * --- | 1,3,5,7 | |
| X | CHEMICAL ABSTRACTS, vol. 72, no. 23, 8 juin 1970, page 337, résumé no. 121378z, Columbus, Ohio, US; & JP-A-70 02 381 (TAKEDA CHEMICAL INDUSTRIES LTD) 27-01-1970 * En entier * --- | 1,3,7 | |
| X | CHEMICAL ABSTRACTS, vol. 51, no. 10, 25 mai 1957, colonnes 7323g-7324f, Columbus, Ohio, US; U. TAKEO et al.: "Syntheses of ring-substituted p-alkylephedrines and p-alkylnorephedrines", & PHARM. BULL. (JAPAN) 4, 182-8(1956) * Colonne 7324d * --- | 1,3,5 | DOMAINES TECHNIQUES RECHERCHES (Int. Cl.4) |
| X | RECUEIL TRAV. CHIM. PAYS-BAS, vol. 68, 1949, pages 781-788, La Haye, NL; NG. PH. BUU-HOI et al.: "Sur quelques composés hétérocycliques renfermant du fluor" * Page 785, ligne 5 * --- | 1,3,5,7 | |
| X | ARCHIV DER PHARMAZIE, vol. 296, no. 5, 1963, pages 324-336; H. OELSCHLÄGER et al.: "Katalytische hydrierungen von 3-Acyl-6-fluor-nitrobenzolen zu 3-Alkyl-6-fluor-anilinen" * Page 332 * --- -/- | 1,3,5,7 | |

Le présent rapport a été établi pour toutes les revendications

| Lieu de la recherche | Date d'achèvement de la recherche | Examinateur |
|---|---|---|
| LA HAYE | 23-08-1988 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

Office européen des brevets

# RAPPORT DE RECHERCHE EUROPEENNE

Numero de la demande

EP 88 40 1149

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 41, no. 2, 20 janvier 1947, colonnes 420f-421e, Columbus, Ohio, US; B.L. ZENITZ et al.: "Amino alcohols. XVI. Phenyl halogenated propadrines", & J. ORG. CHEM. 11, 444-53(1946)<br>* Colonne 420H; colonne 421 b,c *<br>--- | 1,3,5 | |
| X | CHEMICAL ABSTRACTS, vol. 54, no. 6, 25 mars 1960, colonnes 5518g-5519f, Columbus, Ohio, US; K. FUKUI et al.: "Synthesis of fluorine-containing toluenes and their derivatives", & IBID 1428-32<br>* Colonne 5519c *<br>--- | 1,3,5,7 | |
| X | CHEMICAL ABSTRACTS, vol. 63, no. 3, 2 août 1965, colonnes 2951g-2952a, Columbus, Ohio, US; N. SHARGHI et al.: "Some novel ketones and quinolines. Intermediates 2,4,6-tris-(m-trifluoromethylphenyl)-s-triazine, bis(m-trifluoromethyl)-di-benzamide, and tris(m-trifluoromethyl)tribenzamide", & J. CHEM. ENG. DATA 10(2), 196-9(1965)<br>* Colonnes 2951g-2952a * & 7TH COLLECTIVE INDEX, pages 23859s, 4338s<br>--- -/- | 1,3,5 | DOMAINES TECHNIQUES RECHERCHES (Int. Cl.4) |

Le présent rapport a été établi pour toutes les revendications

| Lieu de la recherche | Date d'achèvement de la recherche | Examinateur |
|---|---|---|
| LA HAYE | 23-08-1988 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 88 40 1149

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | J. CHEM. SOC., 1956, pages 2784-2789, Londres, GB; M.A. THOROLD ROGERS: "Aliphatic hydroxylamines. Part III. Reaction with diazotised aromatic amines. A novel synthesis of acylbenzenes from the corresponding amine" * Page 2786, tableau I * --- | 1,3,5,7 | |
| X | US-A-3 708 591 (H.O. BAYER) * Colonne 1, lignes 39-53 * --- | 1,3,6,7 | |
| A | US-A-3 753 680 (H. TILLES) * Colonne 1, lignes 6-34 * --- | 1 | |
| A | US-A-3 654 294 (R. & L. MOLECULAR RESEARCH LTD) * Colonnes 29,30, exemple 9, no. 1-3 * --- | 1 | |
| A | FR-A-2 526 424 (EGYT GYOGYSZERVEGYESZETI GYAR) * Revendications * --- | 1 | DOMAINES TECHNIQUES RECHERCHES (Int. Cl.4) |
| A | JOURNAL OF THE CHEMICAL SOCIETY, PERKIN I, 1981, pages 349-355, Londres, GB; W.T. FLOWERS et al.: "Unsaturated compounds containing nitrogen. Part 4.1 Further reactions of 1-chloro-2,3-diazabutadienes with S-nucleophiles" * Page 350, schéma 2; pages 353-354 * --- -/- | 1 | |

Le présent rapport a été établi pour toutes les revendications

| Lieu de la recherche | Date d'achèvement de la recherche | Examinateur |
|---|---|---|
| LA HAYE | 23-08-1988 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 88 40 1149

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | JOURNAL OF MEDICINAL CHEMISTRY, vol. 14, no. 10, 1971, pages 1017-1020, Washington, D.C., US; A. TAJANA et al.: "Aldehyde disubstituted aminoacethydrazones as potential hypertensive agents" --- | 1 | |
| D,A | IL FARMACO ED. SC., vol. 36, fasc. 4, 1981, pages 269-273, Pavia, IT; M. DE NARDO: "Fusaric acid derivatives and analogues as possible antihypertensive drugs" * Page 272, tableau II; pages 270,273 * --- | 1 | |
| D,A | IL FARMACO ED. SC., vol. 33, fasc. 12, 1978, pages 963-971, Pavia, IT; G. MAZZONE et al.: "Su alcuni aroilidrazoni di alogenobenzaldeidi e 2,5-diaril-1,3,4-ossadiazoli alogeno-sostituiti" * Pages 963,968 * --- | 1,3,4 | DOMAINES TECHNIQUES RECHERCHES (Int. Cl.4) |
| D,A | JOURNAL OF MEDICINAL CHEMISTRY, vol. 20, no. 11, 1977, pages 1520-1521, Washington, D.C., US; J.D. WARREN et al.: "4-substituted semicarbazones of mono- and dichlorobenzaldehydes as antihypertensive agents" * Page 1521, tableau 1 * --- -/- | 1,3,4 | |

Le présent rapport a été établi pour toutes les revendications

| Lieu de la recherche | Date d'achèvement de la recherche | Examinateur |
|---|---|---|
| LA HAYE | 23-08-1988 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 88 40 1149

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | CHEMICAL ABSTRACTS, vol. 86, no. 3, 17 janvier 1977, page 421, résumé no. 16618u, Columbus, Ohio, US; V.B. JIGAJINNI et al.: "Hydrazones of piperidino-N-acetohydrazide and morpholino-N-acetohydrazide of pharmacological interest", & REV. ROUM. CHIM. 1976, 21(8), 1221-5<br>* En entier *<br>--- | 1-4 | |
| P,X | EP-A-0 254 461 (SCHERING AG)<br>* Page 1, ligne 10 - page 5; pages 15-18 *<br>----- | 1,3,6 | |
| | | | DOMAINES TECHNIQUES RECHERCHES (Int. Cl.4) |

Le présent rapport a été établi pour toutes les revendications

| Lieu de la recherche | Date d'achèvement de la recherche | Examinateur |
|---|---|---|
| LA HAYE | 23-08-1988 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)